# EUROPEAN PATENT APPLICATION

(11) **EP 4 810 599 A2**
(43) Date of publication of application: **23.09.2026**
(21) Application number: 26177553.0
(22) Date of filing: 23.04.2021
(51) Int. Cl.: C12N 5/074

(54) **TREATMENT OF VIRUS-INDUCED ACUTE RESPIRATORY DISTRESS SYNDROME**

(30) Priority: 23.04.2020 US 202063014499 P
(62) Document number(s) of the earlier application(s) in accordance with Art. 76 EPC: 21728669.9 / 4 138 868
(71) Applicant: Healios K.K., Tokyo 100-0006 (JP)
(72) Inventor: TING, Anthony E., Shaker Heights, OH 44122 (US); JENKINS, Eric D., Cleveland Heights, OH 44118 (US); VALENTIN-TORRES, Alice, South Euclid, OH 442121 (US)
(74) Representative: Bassil, Nicholas Charles

(57) **Abstract**

The invention is directed to a method of treating viral-induced acute respiratory distress syndrome (ARDS) by administering to a subject with the virial induced ARDS multipotent adult progenitor cells (MAPCs), which are non-embryonic stem, non-germ cells that have a broad differentiation potential and extended replication capacity. The virus inducing the ARDS can be a *Betacoronavirus,* such as, severe acute respiratory syndrome (SARS), Corona Virus or Middle East Respiratory Syndrome (MERS), or severe acute respiratory syndrome Corona Virus 2 (SARS-CoV-2).

## Description

### BACKGROUND

As more information emerges about the current COVID-19 pandemic and its etiology, it is apparent that exacerbated uncontrolled immune responses play a detrimental role in the disease pathology. COVID-19 can cause severe lung inflammation resulting in ARDS. It has been proposed that patients with severe COVID-19 driven ARDS may suffer from a cytokine storm syndrome (Mehta et al., Lancet 2020, 395(10229):1033-1034). In this case, T cell and macrophage driven cytokines such as IL-6, TNFα, IFNγ, IL-2, IL-7, and IL-17 have been reported to be elevated in the plasma of patients with severe COVID-19 when compared to mild COVID-19 cases (Huang et al., Lancet 2020, 395(10223):497-506; Qin et al., Clin. Infect. Dis. 2020, 71(15):762-768; Chen et al., Lancet 2020, 395(10223):507-513). Moreover, CXCL10, CCL2, and CCL3, chemokines involved with the recruitment of T cells and monocyte/macrophages are also increased (Mehta et al., Lancet 2020, 395(10229):1033-1034; Huang et al., Lancet 2020, 395(10223):497-506; Qin et al., Clin. Infect. Dis. 2020, 71(15):762-768). Augmented gene expression of some of these cytokines have also been observed in bronchoalveolar lavage of COVID-19 patients, suggesting increased inflammatory responses in the lungs (Xiong et al., Emerg. Microbes Infect. 2020, 9(1):761-770; Liao et. al., medRxiv 2020, Nature Medicine 2020, 26:842-844).

Analysis of the frequencies of immune cells in the peripheral blood have shown that patients with severe COVID-19 suffer from lymphopenia of both CD4 and CD8 T cells. T cells from these patients express high levels of T cell exhaustion markers such as PD-1 and Tim-3, along with increased expression of activation markers such as CD69 and CD38 (Zhou et al., bioRxiv 2020, doi: https://doi.org/10.1101/2020.02.12.945576). RNA-seq analysis of COVID-19 PBMCs showed increased apoptosis signal pathways, suggesting increased lymphocyte apoptosis may be the cause of lymphopenia (Xiong et al., Emerg. Microbes Infect. 2020, 9(1):761-770). T cell exhaustion results as a response to chronic antigen stimulation and it has been widely described during chronic viral infections. It has been proposed that during COVID-19, T cells are exhausted due to increased and sustained pro-inflammatory responses leading to T cell apoptosis. It was observed that bronchoalveolar CD8 T cells were reduced in patients with severe COVID-19 along with limited clonal expansion compared to patients with mild disease suggesting an impaired CD8 T cell response. Excessive lymphopenia in these patients also resulted in reduced regulatory T cells which contributes to the uncontrolled inflammatory responses observed (Qin et al., Clin. Infect. Dis. 2020, 71(15):762-768).

While lymphocytes in the blood are reduced in severe patients, monocytes/macrophages appear to be highly enriched in the lungs of severe COVID-19 patients (Liao et. al., medRxiv 2020, Nature Medicine 2020, 26:842-844). Phenotypical analysis of peripheral blood monocytes from these patients demonstrated that these cells were enlarged, highly activated, and secreted higher levels of pro-inflammatory cytokines than mild COVID-19 and healthy controls (Zhang et al., J. Leukoc. Biol. 2021, 109(1):13-22, MedRxiv 2020). Increased intermediate (CD14⁺ and CD16⁺) and non-classical (CD14⁻ CD16⁺) monocytes have been observed in ICU patients, suggesting that monocyte composition in severe COVID-19 patients is altered (Liao et. al., medRxiv 2020, Nature Medicine 2020, 26:842-844).

### SUMMARY OF THE INVENTION

It is evident that patients suffering from severe COVID-19 have dysregulated hyperinflammatory responses characterized by increased T cell and monocyte/macrophage activation that contribute and may be the cause of the disease pathology. The inventors investigated whether multipotent adult progenitor cells (MAPC) have the ability to modulate uncontrolled immune responses, thereby reestablishing immune homeostasis and promoting tissue repair in virus-induced acute respiratory distress syndrome (ARDS), specifically in COVID-19-induced ARDS. The inventors used a preparation designated "MultiStem^{®}," which is a commercial preparation based on MAPCs that were disclosed in Jiang et al., Nature 2002, 418:41-9. There is evidence demonstrating that MultiStem^{®} cells reduce dysregulated prolonged T cell activation and proliferation (Reading et al., J. Immunol. 2013, 190:4542-4552; Reading et al., Molecular Therapy 2015, 23(11):1783-1793; Yang et al., Stem Cells 2017, 35:1290-1302; Carty et al., Front. Immunol. 2018, 9:645 (doi: 10.3389/fimmu.2018.00645)). Based on that the inventors considered the possibility of preventing T cell exhaustion (Walker et al., J. Neuroinflammation 2012, 9:228; Reading et al., J. Immunol. 2013, 190(9):4542-4552; Kovacsovics-Bankowski et al., Cell Immunol. 2009, 255(1-2):55-60) by administering MultiStem^{®} to patients with moderate to severe ARDS. Additionally, there are reports that MultiStem^{®} cells promote the differentiation of regulatory T cells (Walker et al., J. Neuroinflamrrcation 2012, 9:228; Reading et al., J. Immunol. 2013, 190(9):4542-4552; Reading et al., Mol. Ther. 2015, 23(11):1783-1793; Yang et al., Stem Cells 2017, 35(5):1290-1302). So the inventors considered that administering MultiStem^{®} might alleviate ARDS and drive the proliferation of AT2 cells (Mock et al., Mucosal Immunol. 2014, 7(6):1440-1451). Furthermore, MultiStem^{®} cells have been reported to reduce monocyte/macrophage proinflammatory profile by decreasing pro-inflammatory cytokine production and expression of pro-inflammatory markers (Walker et al., J. Neuroinflammation 2012, 9:228; DePaul et al., Sci. Rep. 2015, 5:16795; Busch et al., J. Neurosci. 2011, 31(3): 944-953) and to induce the differentiation of M2 anti-inflammatory monocyte/macrophages, thereby promoting lung tissue repair by clearing pathogens and cellular debris while limiting inflammatory responses (Walker et al., J. Neuroinflammation 2012, 9:228; DePaul et al., Sci. Rep. 2015, 5:16795; Busch et al., J. Neurosci. 2011, 31(3): 944-953). The inventors, therefore, investigated whether MultiStem^{®} immunomodulatory properties would help patients suffering from moderate to severe COVID-19 to reduce their dysregulated immune responses by resolving inflammation, restoring homeostasis, and promoting tissue repair.

### DETAILED DESCRIPTION

In one embodiment, a method of treating viral-induced ARDS comprises administering to a subject with viral-induced ARDS MAPCs in an amount sufficient, for a time sufficient, and by a route effective to treat the ARDS.

In one embodiment, the virus that induced the ARDS is *Betacoronavirus.*

In one embodiment, the virus that induced the ARDS is severe acute respiratory syndrome (SARS) coronavirus or Middle East respiratory syndrome (MERS) coronavirus or severe acute respiratory syndrome coronavirus 2 (SARS-CoV-2).

In one embodiment, the subject is human.

In one embodiment, the parameters that are measured in the subject from initial dose are the following: mortality at Day 28 and Day 60; ventilator-free days through Day 28; alive and ventilator-free through 28 days, which incorporates death and days free of invasive mechanical ventilation; percentage of subjects who are alive and off the ventilator at Days 7 and 28; change in Sequential Organ Failure Assessment score from Day 0 (pre-infusion) through Day 3 and Day 7; ventilator-free days from Day 0 through Day 60; ICU-free days from Day 0 through Day 28 and Day 60; white blood cell populations on Days 0, 3, and 7; inflammatory biomarkers on Days 0, 1, 3, and 7; and changes in levels of oxygenation (PaO2/FiO2 ratio), oxygenation index, peak and plateau pressures, and PEEP requirements from baseline (Day 0) through Days 1, 2, 3, and 7 (and Days 4, 5, and 6 for subjects receiving 2 doses of MAPC). These could be relative to the untreated population (i.e., no MAPC infusion) - based on known historical averages.

In one embodiment, the dosage regimen is 900 million to 1.2 billion MAPC cells provided at Day 0 or in some cases, Day 0 and an additional dose of 900 million to 1.2 billion MAPC cells 72 to 96 hours after the first dose.

In one embodiment, the route of administration is intravenous.

In one embodiment, the MAPCs are allogeneic.

In one embodiment, the MAPCs are derived from bone marrow.

In one embodiment, the MAPCs are human.
1. Viruses treatable - influenza, coronavirus including (SARS-CoV, MERS-CoV, SARS-CoV2), herpes simplex virus, cytomegalovirus, rhinoviruses, respiratory syncytial virus, parainfluenza virus, human metapneumovirus, adenovirus, and viruses that can cause serious systemic illness (viral hemorrhagic fevers) that can be complicated by ARDS resulting from direct endothelial damage or indirectly through cytokine storm / sepsis syndrome, such as filoviruses (e.g., Ebola, Marburg), Arenaviruses (LCMV, Lassa, Junin, Lujo, etc.), Bunyaviruses (Rift Valley fever and Crimean-Congo hemorrhagic fever viruses, and Hantaviruses), and Flaviviruses (yellow fever, Dengue fever, Japanese encephalitis, West Nile viruses, Zika virus, etc.).
2. Acute respiratory distress syndrome (ARDS) - a severe form of inflammatory lung injury characterized by increased vascular permeability in the lung. Clinically, ARDS is defined by the presence of severe hypoxemia and bilateral opacities on chest imaging not fully explained by cardiac failure or fluid overload. Specifically, it is defined by the Berlin Definition (or Kigali modification of the Berlin Criteria (see, *e.g.,* Riviello et al., Am. J. Respir. Crit. Care Med. 2016, 193(1):52-59)).
3. Berlin (and Kigali modification) definitions of ARDS -
   a. Timing - the onset must be within 1 week of a known clinical insult or new or worsening respiratory symptom.
   b. Imaging (Chest radiograph or computed tomography scan or ultrasound (Kigali modification))- bilateral opacities consistent with pulmonary edema on the chest radiograph.
   c. Origin of edema - respiratory failure not fully explained by cardiac failure or fluid overload as judged by the treating physician using all available data
   d. Oxygenation
      i. Mild ARDS - 200 mm Hg < PaO2/FIO2 ≤ 300 mm Hg with PEEP or CPAP ≥5 cm H2O; or SpO2/FIO2 ≤315 (Kigali modification)
      ii. Moderate ARDS - 100 mm Hg < PaO2/FIO2 ≤ 200 mm Hg with PEEP ≥5 cm H2O
      iii. Severe ARDS - PaO2/FIO2 ≤ 100 mm Hg with PEEP ≥5 cm H2O
         1. Abbreviations: CPAP, continuous positive airway pressure; FIO2, fraction of inspired oxygen; PaO2, partial pressure of arterial oxygen; PEEP, positive end-expiratory pressure.
4. In one embodiment, patients may be selected as follows: Dysregulated immune response - a maladaptive change in molecular control of immune system processes that in the case of ARDS leads to disruption of the pulmonary alveolar-capillary barrier, resulting in lung injury characterized by hypoxemia, inflammation, and noncardiogenic pulmonary edema.
5. In one embodiment, patients may be selected as follows: Worsening ARDS -a PaO2/FiO2 ≤ 300 mmHg on PEEP ≥5 cm H2O; and PaO2/FiO2 remains <200 mmHg or PaO2/FiO2 has not increased by more than 100 mmHg from the screening assessment to PaO2/FiO2 measured within 6 hours of IP administration for the first dose, or from Day 0 baseline to PaO2/FiO2 measured within 6 hours of administration of a second dose of IP.

In other embodiments, other parameters may be addressed and / or measured. Applicant has various endpoints on which the MAPCs may have a beneficial effect. Any of these endpoints could be measured pre and / or post treatment with the MAPCs. Thus, MAPCs may have a beneficial effect on the uncontrolled immune responses that play a detrimental role in the disease pathology. The following are the various endpoints: reduce lung inflammation; reduce cytokine storm syndrome; reduce T cell and macrophage driven cytokines, such as IL-6, TNF-α, IFNγ, IL-2 and IL-7; reduce CXCL10, CCL2 and CCL3, which are cytokines that arc involved in the recruitment of T cells and monocytes / macrophages; reduce monocyte recruitment and / or activation; reduce inflammatory responses in the lungs; reduce lymphopenia of CD4 and / or CD8 T cells; reduce T cell exhaustion markers, such as PD-1 and TIM-3 such that T cell exhaustion itself is reduced; reduce expression of activation markers, such as CD69 and CD38; reduce T cell apoptosis; have an effect on apoptosis signal pathways; affect monocytes that in COVID patients are enlarged, highly activated and secrete levels of pro-inflammatory cytokines; decrease intermediate (CD14- and CD16+) and non-classical (CD14- and CD16+) monocytes, increase T regulatory cells, and decrease activation of monocytes and secretion of pro-inflammatory cytokines from those monocytes. Accordingly, any of these endpoints could be measured pre and / or post MAPC treatment. When measured post treatment, MAPCs may affect these parameters as described compared to historical averages in patients that do not manifest ARDS, and possibly patients with ARDS but wherein the ARDS is not virus-induced.

Cells include, but are not limited to, cells that are not embryonic stem cells and not germ cells, having some characteristics of embryonic stem cells, but being derived from non-embryonic tissue, and providing the effects described in this application. The cells may naturally achieve these effects (i.e., not genetically or pharmaceutically modified). However, natural expressors can be genetically or pharmaceutically modified to increase potency. In one embodiment, the stem cells can be non-HLA matched, allogeneic cells.

The cells may express pluripotency markers, such as oct4. They may also express markers associated with extended replicative capacity, such as telomerase. Other characteristics of pluripotency can include the ability to differentiate into cell types of more than one germ layer, such as two or three of ectodermal, endodermal, and mesodermal embryonic germ layers. The cells may be highly expanded without being transformed or tumorigenic and also maintain a normal karyotype. In one embodiment, the non-embryonic stem, non-germ cells may have undergone a desired number of cell doublings in culture. For example, non-embryonic stem, non-germ cells may have undergone at least 10-40 cell doublings in culture, such as 30-35 cell doublings, wherein the cells are not transformed and have a normal karyotype. The cells may differentiate into at least one cell type of each of two of the endodermal, ectodermal, and mesodermal embryonic lineages and may include differentiation into all three. Further, the cells may not be tumorigenic, such as, not producing teratomas. If cells are transformed or tumorigenic, and it is desirable to use them for infusion, such cells may be disabled so they cannot form tumors in vivo, as by treatment that prevents cell proliferation into tumors. Such treatments are well known in the art.

Cells include, but are not limited to, the following numbered embodiments:
1. Isolated expanded non-embryonic stem, non-germ cells, the cells having undergone at least 10-40 cell doublings in culture, wherein the cells express oct4, are not transformed, and have a normal karyotype.
2. The non-embryonic stem, non-germ cells of 1 above that further express one or more of telomerase, rex-1, or sox-2.
3. The non-embryonic stem, non-germ cells of 1 above that can differentiate into at least one cell type of at least two of the endodermal, ectodermal, and mesodermal embryonic lineages.
4. The non-embryonic stem, non-germ cells of 3 above that further express one or more of telomerase, rox-1, or sox-2.
5. The non-embryonic stem, non-germ cells of 3 above that can differentiate into at least one cell type of each of the endodermal, ectodermal, and mesodermal embryonic lineages.
6. The non-embryonic stem, non-germ cells of 5 above that further express one or more of telomerase, rex-1, or sox-2.
7. Isolated expanded non-embryonic stem, non-germ cells that are obtained by culture of non-embryonic, non-germ tissue, the cells having undergone at least 40 cell doublings in culture, wherein the cells are not transformed and have a normal karyotype.
8. The non-embryonic stem, non-germ cells of 7 above that express one or more of oct4, telomerase, rex-1, or sox-2.
9. The non-embryonic stem, non-germ cells of 7 above that can differentiate into at least one cell type of at least two of the endodermal, ectodermal, and mesodermal embryonic lineages.
10. The non-embryonic stem, non-germ cells of 9 above that express one or more of oct4, telomerase, rex-1, or sox-2.
11. The non-embryonic stem, non-germ cells of 9 above that can differentiate into at least one cell type of each of the endodermal, ectodermal, and mesodermal embryonic lineages.
12. The non-embryonic stem, non-germ cells of 11 above that express one or more of oct4, telomerase, rex-1, or sox-2.
13. Isolated expanded non-embryonic stem, non-germ cells, the cells having undergone at least 10-40 cell doublings in culture, wherein the cells express telomerase, are not transformed, and have a normal karyotype.
14. The non-embryonic stem, non-germ cells of 13 above that further express one or more of oct4, rex-1, or sox-2.
15. The non-embryonic stem, non-germ cells of 13 above that can differentiate into at least one cell type of at least two of the endodermal, ectodermal, and mesodermal embryonic lineages.
16. The non-embryonic stem, non-germ cells of 15 above that further express one or more of oct4, rex-1, or sox-2.
17. The non-embryonic stem, non-germ cells of 15 above that can differentiate into at least one cell type of each of the endodermal, ectodermal, and mesodermal embryonic lineages.
18. The non-embryonic stem, non-germ cells of 17 above that further express one or more of oct4, rex-1, or sox-2.
19. Isolated expanded non-embryonic stem, non-germ cells that can differentiate into at least one cell type of at least two of the endodermal, ectodermal, and mesodermal embryonic lineages, said cells having undergone at least 10-40 cell doublings in culture.
20. The non-embryonic stem, non-germ cells of 19 above that express one or more of oct4, telomerase, rex-1, or sox-2.
21. The non-embryonic stem, non-germ cells of 19 above that can differentiate into at least one cell type of each of the endodermal, ectodermal, and mesodermal embryonic lineages.
22. The non-embryonic stem, non-germ cells of 21 above that express one or more of oct4, telomerase, rex-1, or sox-2.

The cells lack expression of HLA-DR, CD45, glyA, and CD34. The cells may express one or more of CD90, CD49c, CD13, CD10, and CD29.

The cells may be derived from bone marrow, such as, human bone marrow.

Since the stem cells may provide the effects described herein by means of secreted molecules, the various embodiments described herein for administration of stem cells may be done by administration of one or more of the secreted molecules, such as might be in conditioned culture medium. In one embodiment, a conditioned medium is used instead of the stem cells. Components of the medium can be separated from culture medium components, such as, to eliminate animal serum.

The stem cells may be prepared by the isolation and culture conditions described herein. In a specific embodiment, they are prepared by culture conditions that are described herein involving lower oxygen concentrations combined with higher serum.

It should be understood that this invention is not limited to the particular methodology, protocols, and reagents, etc., described herein and, as such, may vary. The terminology used herein is for the purpose of describing particular embodiments only, and is not intended to limit the scope of the disclosed invention, which is defined solely by the claims.

The section headings are used herein for organizational purposes only and are not to be construed as in any way limiting the subject matter described.

The methods and techniques of the present application are generally performed according to conventional methods well-known in the art and as described in various general and more specific references that are cited and discussed throughout the present specification unless otherwise indicated. See, *e.g.,* Sambrook et al., Molecular Cloning: A Laboratory Manual, 3rd Ed., Cold Spring Harbor Laboratory Press, Cold Spring Harbor, N.Y. (2001) and Ausubel et al., Current Protocols in Molecular Biology, Greene Publishing Associates (1992), and Harlow and Lane, Antibodies: A Laboratory Manual, Cold Spring Harbor Laboratory Press, Cold Spring Harbor, N.Y. (1990).

### DEFINITIONS

"A" or "an" means herein one or more than one; at least one. Where the plural form is used herein, it generally includes the singular.

A "cell bank" is industry nomenclature for cells that have been grown and stored for future use. Cells may be stored in aliquots. They can be used directly out of storage or may be expanded after storage. This is a convenience so that there are "off the shelf" cells available for administration. The cells may already be stored in a pharmaceutically-acceptable excipient so they may be directly administered or they may be mixed with an appropriate excipient when they are released from storage. Cells may be frozen or otherwise stored in a form to preserve viability. In one embodiment of the invention, cell banks are created in which the cells have been selected for enhanced potency to achieve the effects described in this application. Following release from storage, and prior to administration, it may be preferable to again assay the cells for potency. This can be done using any of the assays, direct or indirect, described in this application or otherwise known in the art. Then cells having the desired potency can then be administered. Banks can be made using autologous cells (derived from the organ donor or recipient). Or banks can contain cells for allogeneic uses.

"Co-administer" with respect to this invention means to administer together two or more agents. Within the context of the present invention, in one embodiment the stem cells are administered in combination with other treatment modalities, such as convalescent plasma; anti-viral agents including remdesivir, favipiravir, or kaletra, a combination of lopinavir and ritonavir; high dose vitamin C; or IL-6 inhibitors including tocilizumab, siltuximab or sarilumab.

"Comprising" means, without other limitation, including the referent, necessarily, without any qualification or exclusion on what else may be included. For example, "a composition comprising x and y" encompasses any composition that contains x and y, no matter what other components may be present in the composition. Likewise, "a method comprising the step of x" encompasses any method in which x is carried out, whether x is the only step in the method or it is only one of the steps, no matter how many other steps there may be and no matter how simple or complex x is in comparison to them. "Comprised of and similar phrases using words of the root "comprise" are used herein as synonyms of "comprising" and have the same meaning.

"Comprised of" is a synonym of "comprising" (see above).

"Effective amount" generally means an amount which achieves the specific desired effects described in this application. For example, an effective amount is an amount sufficient to effectuate a beneficial or desired clinical result. Within the context of this invention generally the desired effect is a clinical improvement compensating for the ineffective or pathological function in a subject. The effective amounts can be provided all at once in a single administration or in fractional amounts that provide the effective amount in several administrations. The precise determination of what would be considered an effective amount may be based on factors individual to each subject, including the severity of the disease/deficiency, health of the patient, age, etc. One skilled in the art will be able to determine the effective amount based on these considerations which are routine in the art. As used herein, "effective dose" means the same as "effective amount."

Accordingly, an effective amount is that in which the clinical symptoms of the subject are improved. So, as a non-limiting example, an effective amount of stem cells would be that which is sufficient to results in subjects: being extubated within 28 days of receiving the first dose of MAPC cells; having a PaO2/FiO2 >300 mmHg on PEEP <5 cm H2O by day 28; having ≥ 10% less mortality compared to subjects not receiving the MAPCs, which can be determined based on historical averages; or having a mean increase of ≥ 4 VFD through Day 28 compared to subjects not receiving the MAPCs, which can be determined based on historical averages.

"Effective route" generally means a route which provides for delivery of an agent to a desired compartment, system, or location. For example, an effective route is one through which an agent can be administered to provide at the desired site of action an amount of the agent sufficient to effectuate a beneficial or desired clinical result.

The term "exogenous," when used in relation to a stem cell, generally refers to a stem cell that is external to the subject and which has been exposed to (*e.g.,* contacted with) the islets that are intended for transplantation by an effective route. An exogenous stem cell may be from the same subject or from a different subject. In one embodiment, exogenous stem cells can include stem cells that have been harvested from a subject, isolated, expanded *ex vivo,* and then exposed to the islets intended for transplantation by an effective route.

Use of the term "includes" is not intended to be limiting.

"Increase" or "increasing" means to induce a biological event entirely or to increase the degree of the event.

The term "isolated" refers to a cell or cells which are not associated with one or more cells or one or more cellular components that are associated with the cell or cells *in vivo.* An "enriched population" means a relative increase in numbers of a desired cell relative to one or more other cell types *in vivo* or in primary culture.

However, as used herein, the term "isolated" does not indicate the presence of only the cells of the invention. Rather, the term "isolated" indicates that the cells of the invention are removed from their natural tissue environment and are present at a higher concentration as compared to the normal tissue environment. Accordingly, an "isolated" cell population may further include cell types in addition to the cells of the invention cells and may include additional tissue components. This also can be expressed in terms of cell doublings, for example. A cell may have undergone 10, 20, 30, 40 or more doublings *in vitro* or *ex vivo* so that it is enriched compared to its original numbers *in vivo* or in its original tissue environment (e.g., bone marrow, peripheral blood, placenta, umbilical cord, umbilical cord blood, etc.).

"MAPC" is an acronym for "multipotent adult progenitor cell." It refers to a cell that is not an embryonic stem cell or germ cell but has some characteristics of these. MAPC can be characterized in a number of alternative descriptions, each of which conferred novelty to the cells when they were discovered. They can, therefore, be characterized by one or more of those descriptions. First, they can have extended replicative capacity in culture without being transformed (tumorigenic) and with a normal karyotype. Second, they may give rise to cell progeny of more than one germ layer, such as two or all three germ layers *(i.e.,* endoderm, mesoderm and ectoderm) upon differentiation. Third, although they are not embryonic stem cells or germ cells, they may express markers of these primitive cell types so that MAPCs may express one or more of Oct 3/4 *(i.e.,* Oct4, Oct 3A). Fourth, like a stem cell, they may self-renew, that is, have an extended replication capacity without being transformed. This means that these cells express telomerase *(i.e.,* have telomerase activity). Accordingly, the cell type that was designated "MAPC" may be characterized by alternative basic characteristics that describe the cell via some of its novel properties.

The term "adult" in MAPC is non-restrictive. It refers to a non-embryonic somatic cell as above. MAPCs are karyotypically normal and do not form teratomas *in vivo.* This acronym was first used in U.S. Patent No. 7,015,037 to describe a cell isolated from bone marrow that had extensive replicative capacity and expressed pluripotency markers.

MAPC represents a more primitive progenitor cell population than MSC (Verfaillie, C.M., Trends Cell Biol 12:502-8 (2002), Jahagirdar, B.N., et al., Exp Hematol, 29:543-56 (2001); Reyes, M. and C.M. Verfaillie, Ann N Y Acad Sci, 938:231-233 (2001); Jiang, Y. et al., Exp Hematol, 30896-904 (2002); and Jiang, Y. et al., Nature, 418:41-9 (2002)).

The term "MultiStem^{®}" is the trade name for a cell preparation based on the MAPCs of U.S. Patent No. 7,015,037, i.e., a non-embryonic stem, non-germ cell as described above. MultiStem^{®} is prepared according to cell culture methods disclosed in this patent application, particularly, lower oxygen and higher serum. MultiStem^{®} is highly expandable, karyotypically normal, and does not form teratomas *in vivo.* It may differentiate into cell lineages of more than one germ layer and may express telomerase.

"Pharmaceutically-acceptable carrier" is any pharmaceutically-acceptable medium for the cells and/or islets used in the present invention. Such a medium may retain isotonicity, cell metabolism, pH, and the like. It is compatible with administration to a subject and can be used, therefore, for islet and/or cell delivery and treatment.

"Progenitor cells" are cells produced during differentiation of a stem cell that have some, but not all, of the characteristics of their terminally-differentiated progeny. Defined progenitor cells, such as "cardiac progenitor cells," are committed to a lineage, but not to a specific or terminally differentiated cell type. The term "progenitor" as used in the acronym "MAPC" does not limit these cells to a particular lineage. A progenitor cell can form a progeny cell that is more highly differentiated than the progenitor cell.

The term "reduce" as used herein means to prevent as well as decrease. In the context of treatment, to "reduce" is to either prevent or ameliorate the deficiency. This includes the endpoint parameters described above, including, but not limited to, a reduction of inflammatory biomarkers (IFNgamma, IL1beta, IL6, IL8, TNFalpha, CXCL10, IL10, MCP1, IL-1, IL-2RA, IL-7, RAGE, PD1, IL1-R2) measured over the first 7 days after the initial cell dosing.

"Selecting" a cell with a desired level of potency can mean identifying (as by assay), isolating, and expanding a cell. This could create a population that has a higher potency than the parent cell population from which the cell was isolated. The "parent" cell population refers to the parent cells from which the selected cells divided. "Parent" refers to an actual P1 → F1 relationship (i.e., a progeny cell). So if cell X is isolated from a mixed population of cells X and Y, in which X is an expressor and Y is not, one would not classify a mere isolate of X as having enhanced expression. But, if a progeny cell of X is a higher expressor, one would classify the progeny cell as having enhanced expression.

To select a cell that achieves the desired effect would include both an assay to determine if the cells achieve the desired effect and would also include obtaining those cells. The cell may naturally achieve the desired effect in that the effect is not achieved by an exogenous transgene/DNA. But an effective cell may be improved by being incubated with or exposed to an agent that increases the effect. The cell population from which the effective cell is selected may not be known to have the potency prior to conducting the assay. The cell may not be known to achieve the desired effect prior to conducting the assay. As an effect could depend on gene expression and/or secretion, one could also select on the basis of one or more of the genes that cause the effect.

Selection could be from cells in a tissue. For example, in this case, cells would be isolated from a desired tissue, expanded in culture, selected for achieving the desired effect, and the selected cells further expanded.

Selection could also be from cells *ex vivo,* such as cells in culture. In this case, one or more of the cells in culture would be assayed for achieving the desired effect and the cells obtained that achieve the desired effect could be further expanded.

Cells could also be selected for enhanced ability to achieve the desired effect. In this case, the cell population from which the enhanced cell is obtained already has the desired effect. Enhanced effect means a higher average amount per cell than in the parent population.

The parent population from which the enhanced cell is selected may be substantially homogeneous (the same cell type). One way to obtain such an enhanced cell from this population is to create single cells or cell pools and assay those cells or cell pools to obtain clones that naturally have the enhanced (greater) effect (as opposed to treating the cells with a modulator that induces or increases the effect) and then expanding those cells that are naturally enhanced.

However, cells may be treated with one or more agents that will induce or increase the effect. Thus, substantially homogeneous populations may be treated to enhance the effect.

If the population is not substantially homogeneous, then, it is preferable that the parental cell population to be treated contains at least 100 of the desired cell type in which enhanced effect is sought, more preferably at least 1,000 of the cells, and still more preferably, at least 10,000 of the cells. Following treatment, this sub-population can be recovered from the heterogeneous population by known cell selection techniques and further expanded if desired.

Thus, desired levels of effect may be those that are higher than the levels in a given preceding population. For example, cells that are put into primary culture from a tissue and expanded and isolated by culture conditions that are not specifically designed to produce the effect may provide a parent population. Such a parent population can be treated to enhance the average effect per cell or screened for a cell or cells within the population that express greater degrees of effect without deliberate treatment. Such cells can be expanded then to provide a population with a higher (desired) expression.

"Self-renewal" of a stem cell refers to the ability to produce replicate daughter stem cells having differentiation potential that is identical to those from which they arose. A similar term used in this context is "proliferation."

"Subject" means a vertebrate, such as a mammal, such as a human. Mammals include, but are not limited to, humans, dogs, cats, horses, cows, and pigs.

The term "therapeutically effective amount" refers to the amount of an agent determined to produce any therapeutic response in a mammal. For example, effective therapeutic agents may prolong the survivability of the patient, and/or inhibit overt clinical symptoms. Treatments that are therapeutically effective within the meaning of the term as used herein, include treatments that improve a subject's quality of life even if they do not improve the disease outcome *per se.* Such therapeutically effective amounts are readily ascertained by one of ordinary skill in the art. Thus, to "treat" means to deliver such an amount. Thus, treating can prevent or ameliorate any pathological symptoms. In one aspect, treatment means to improve lung function as measured by PaO2, i.e., towards or in normal ranges (in this case, can be relative to untreated patients - via historical averages).

In the context of the invention a therapeutically effective amount is that amount of stem cells that results in an improvement in the clinical outcome.

The term "therapeutically effective time" can refer to the time necessary to achieve the clinical improvement.

A therapeutically effective time could also refer to the time required for a subject to achieve an improved clinical status. In the present case, cells could be delivered within 48 hours of diagnosis of ARDS and evaluated over a 28-day period.

The term "therapeutically effective route" refers to the routes of administration that may be effective for achieving an improved clinical outcome. This includes intravenous delivery either by a peripheral or central line.

An appropriate amount of stem cells to achieve the beneficial effects is determined empirically. A dose range could be 900,000-1,200,000 stem cells with the possibility of a repeat dose provided 72 to 96 hours after the first dose. Thus, these amounts need to be determined empirically based on the method of delivery, the severity of the illness, and the like.

"Treat," "treating," or "treatment" are used broadly in relation to the invention and each such term encompasses, among others, preventing, ameliorating, inhibiting, or curing a deficiency, dysfunction, disease, or other deleterious process, including those that interfere with and/or result from a therapy.

A "sufficient time" and a "sufficient amount" shall have the same meanings as an "effective time" and an "effective amount," respectively. Clinical symptoms that can be assessed to ascertain whether the cells to which this application is directed are administered in sufficient amounts, for sufficient time and by an effective route include one or more of the following:
a. reduction in lung inflammation;
b. reduction in T cell and macrophage-driven cytokines;
c. reduction in chemokines that are involved with the recruitment of T cells and monocytes / macrophages; increase in CD4 and CD8 T cells;
d. decreased lymphopenia, including reduced levels of T cell exhaustion markers, reduced expression of T cell activation markers, reduced apoptosis signal pathways, reversal of T cell exhaustion, reduced T cell apoptosis, increased CD8 T cell counts, increased T regulatory cells, reduction of monocytes / macrophages in the lungs, phenotypic changes of peripheral blood monocytes, decreased intermediate and non-classical monocytes, differentiation of regulatory T cells, proliferation of AT2 cells, decreased pro-inflammatory cytokine production, decreased expression of pro-inflammatory markers, increased differentiation of M2 anti-inflammatory monocytes / macrophages, clearance of pathogens and cellular debris in lung tissue;
e. other parameters that can be measured to determine the efficacy of the cells in treating the patient, including mortality, ventilator-free days, changes in sequential organ failure, ICU-free days, changes of levels of oxygenation, oxygenation index, peak and plateau pressures and PEEP requirements from baseline, which can be relative to the untreated population; self-(or surrogate-) reported quality of life, such as, via a survey instrument like EQ-50 may also be assessed.
f. other parameters include decreased vascular permeability in the lung, decreased hypoxemia and bilateral opacities on chest imaging, decreased non-cardiogenic pulmonary edema, reduced respiratory failure, positive airway pressure, fraction of inspired oxygen, partial pressure of arterial oxygen, and positive end-point expiratory pressure; imaging scoring systems evaluating degree of lung consolidation, infiltration, bronchiectasis, fibrosis or functional lung volumes;
g. other parameters include reduction in cytokine storm syndrome.

"Validate" means to confirm. In the context of the invention, one confirms that a cell has a desired potency for beneficially affecting the subject. This is so that one can then use that cell (in treatment, banking, drug screening, etc.) with a reasonable expectation of efficacy. Accordingly, to validate means to confirm that the cells, having been originally found to have/established as having the desired activity, in fact, retain that activity. Thus, validation is a verification event in a two-event process involving the original determination and the follow-up determination. The second event is referred to herein as "validation."

The present invention can be practiced, preferably, using stem cells of vertebrate species, such as humans, non-human primates, domestic animals, livestock, and other non-human mammals. These include, but are not limited to, those cells described below.

### Transcription Factors

A number of transcription factors and exogenous cytokines have been identified that influence the potency status of stem cells *in vivo.* The first transcription factor to be described that is involved in stem cell pluripotency is Oct4. Oct4 belongs to the POU (Pit-Oct-Unc) family of transcription factors and is a DNA binding protein that is able to activate the transcription of genes, containing an octameric sequence called "the octamer motif" within the promoter or enhancer region. Oct4 is expressed at the moment of the cleavage stage of the fertilized zygote until the egg cylinder is formed. The function of Oct3/4 is to repress differentiation inducing genes *(i.e.,* FoxaD3, hCG) and to activate genes promoting pluripotency (FGF4, Utf1, Rex1). Sox2, a member of the high mobility group (HMG) box transcription factors, cooperates with Oct4 to activate transcription of genes expressed in the inner cell mass. It is essential that Oct3/4 expression in embryonic stem cells is maintained between certain levels. Over-expression or down-regulation of >50% of Oct4 expression level will alter embryonic stem cell fate, with the formation of primitive endoderm/mesoderm or trophectoderm, respectively. *In vivo,* Oct4 deficient embryos develop to the blastocyst stage, but the inner cell mass cells are not pluripotent. Instead they differentiate along the extraembryonic trophoblast lineage. Sall4, a mammalian Spalt transcription factor, is an upstream regulator of Oct4, and is therefore important to maintain appropriate levels of Oct4 during early phases of embryology. When Sall4 levels fall below a certain threshold, trophectodermal cells will expand cctopically into the inner cell mass. Another transcription factor required for pluripotency is Nanog, named after a Celtic tribe "Tir Nan Og": the land of the ever young. *In vivo,* Nanog is expressed from the stage of the compacted morula, is subsequently defined to the inner cell mass and is downregulated by the implantation stage. Downregulation of Nanog may be important to avoid an uncontrolled expansion of pluripotent cells and to allow multilineage differentiation during gastrulation. Nanog null embryos, isolated at day 5.5, consist of a disorganized blastocyst, mainly containing extraembryonic endoderm and no discernible epiblast.

### Isolation and Growth of MAPCs

Methods of MAPC isolation are known in the art. See, for example, U.S. Patent 7,015,037, and these methods, along with the characterization (phenotype) of MAPCs, are incorporated herein by reference. MAPCs can be isolated from multiple sources, including, but not limited to, bone marrow, placenta, umbilical cord and cord blood, muscle, brain, liver, spinal cord, blood or skin. It is, therefore, possible to obtain bone marrow aspirates, brain or liver biopsies, and other organs, and isolate the cells using positive or negative selection techniques available to those of skill in the art, relying upon the genes that are expressed (or not expressed) in these cells (*e.g.,* by functional or morphological assays such as those disclosed in the above-referenced applications, which have been incorporated herein by reference).

MAPCs have also been obtained by modified methods described in Breyer et al., Experimental Hematology, 34:1596-1601 (2006); Subramanian et al. (S. Ding (ed.)), Methods Mol. Biol., 636:55-78 (2010); Boozer et al., J. Stem Cells 4(1):17-28 (2009); and Vaes et al., Methods Mol. Biol.,1235:49-58 (2015), incorporated by reference for these methods.

### MAPCs from Human Bone Marrow as Described in U.S. Patent 7,015,037

MAPCs do not express CD45 or glycophorin-A (Gly-A). The mixed population of cells was subjected to a Ficoll Hypaque separation. The cells were then subjected to negative selection using anti-CD45 and anti-Gly-A antibodies, depleting the population of CD45+ and Gly-A+ cells, and the remaining approximately 0.1% of marrow mononuclear cells were then recovered. Cells could also be plated in fibronectin-coated wells and cultured as described below for 2-4 weeks to deplete the cells of CD45+ and Gly-A+ cells. In cultures of adherent bone marrow cells, many adherent stromal cells undergo replicative senescence around cell doubling 30 and a more homogenous population of cells continues to expand and maintains long telomeres.

### Additional Culture Methods

In additional experiments, the density at which MAPCs are cultured can vary from about 100 cells/cm² or about 150 cells/cm² to about 10,000 cells/cm², including about 200 cells/cm² to about 1500 cells/cm² to about 2000 cells/cm². The density can vary between species. Additionally, optimal density can vary depending on culture conditions and source of cells. It is within the skill of the ordinary artisan to determine the optimal density for a given set of culture conditions and cells.

Also, effective atmospheric oxygen concentrations of less than about 10%, including about 1-5% and, especially, 3-5%, can be used at any time during the isolation, growth and differentiation of MAPCs in culture.

Cells may be cultured under various serum concentrations, *e.g.*, about 2-20%. Fetal bovine serum may be used. Higher serum may be used in combination with lower oxygen tensions, for example, about 15-20%. Cells need not be selected prior to adherence to culture dishes. For example, after a Ficoll gradient, cells can be directly plated, *e.g.,* 250,000-500,000/cm². Adherent colonies can be picked, possibly pooled, and expanded.

In one embodiment, used in the experimental procedures in the Examples, high serum (around 15-20%) and low oxygen (around 3-5%) conditions were used for the cell culture. Specifically, adherent cells from colonies were plated and passaged at densities of about 1700-2300 cells/cm² in 18% serum and 3% oxygen (with PDGF and EGF).

In an embodiment specific for MAPCs, supplements are cellular factors or components that allow MAPCs to retain the ability to differentiate into cell types of more than one embryonic lineage, such as all three lineages. This may be indicated by the expression of specific markers of the undifferentiated state, such as Oct 3/4 (Oct 3A) and/or markers of high expansion capacity, such as telomerase.

Serum may provide a significant source of variability. The optimum serum concentrations can vary depending on serum batch characteristics. Accordingly, different serum lots are screened for their capacity to support optimal MAPC expansion. A large quantity of serum from an appropriate batch can be reserved. Ideally, MAPC are seeded at densities between and 200 and 2,000 cells per cm² and higher densities are avoided. They are constantly passaged at sub-confluency (30 -70%). Using these conditions the MAPCs can be routinely expanded for up to 15 to 20 passages (50-70 population doublings).

### Pharmaceutical Formulations

In certain embodiments, the cell populations arc present within a composition adapted for and suitable for delivery, i.e., physiologically compatible.

In some embodiments the purity of the cells for administration with or to the islets is about 100% (substantially homogeneous). In other embodiments it is 95% to 100%. In some embodiments it is 85% to 95%. Particularly, in the case of admixtures with other cells, the percentage can be about 10%-15%, 15%-20%, 20%-25%, 25%-30%, 30%-35%, 35%-40%, 40%-45%, 45%-50%, 60%-70%, 70%-80%, 80%-90%, or 90%-95%. Or isolation/purity can be expressed in terms of cell doublings where the cells have undergone, for example, 10-20, 20-30, 30-40, 40-50 or more cell doublings.

### Dosing

Doses (i.e., the number of cells) for humans or other mammals can be determined without undue experimentation by the skilled artisan, from this disclosure, the documents cited herein, and the knowledge in the art. The optimal dose to be used in accordance with various embodiments of the invention will depend on numerous factors, including the following: the disease being treated and its stage; the species of the donor, their health, gender, age, weight, and metabolic rate; the donor's immunocompetence; other therapies being administered; and expected potential complications from the donor's history or genotype. The parameters may also include: whether the cells are syngeneic, autologous, allogeneic, or xenogeneic; their potency; the site and/or distribution that must be targeted; and such characteristics of the site such as accessibility to cells. Additional parameters include coadministration with other factors (such as growth factors and cytokines). The optimal dose in a given situation also will take into consideration the way in which the cells are formulated, the way they are administered (e.g., perfusion, intra-organ, etc.), and the degree to which the cells will be localized at the target sites following administration.

### EXAMPLE

Details for isolating and expanding MAPCs are found above.

The population for this study is men and women 18 to 89 years of age with a diagnosis of moderate to severe ARDS, as defined by the Berlin definition. All diagnostic criteria for moderate to severe ARDS are confirmed to be present within a 24-hour period. The 48-hour period permitted for commencing infusion of MultiStem^{®} or placebo starts once the last ARDS diagnosis criterion is met. Subjects are confirmed to have persistent or worsening ARDS, confirmed by PaO2/FiO2 measured within the 6 hours prior to randomization. All subjects receive either MultiStem^{®} therapy (900 million or 1.2 billion cells per dose) or placebo by intravenous (IV) infusion within 48 hours of meeting the last ARDS diagnosis criterion.

Cohorts enrolled as follows:
❖ Cohort 1
   ➢ Cohort 1a (MultiStem^{®} product): 3 subjects treated open-label with 900 million MultiStem^{®} cells per dose.
   ➢ Cohort 1b (MultiStem^{®} product): 3 subjects treated open-label with 1.2 billion MultiStem^{®} cells per dose.
   ➢ Cohort 1c (MultiStem^{®} product): 3 subjects treated open-label with 900 million or 1.2 billion MultiStem^{®} cells per dose. The dose will be selected based on review of data from Cohorts 1a and 1b by the DSMB.
   ➢ Cohort 1d (MultiStem^{®} product): 3 (or more) subjects treated open-label with 900 million or 1.2 billion MultiStem^{®} cells per dose. Subjects meeting specific criteria of persistent or worsening ARDS will be eligible to receive an identical second dose of 900 million or 1.2 billion MultiStem^{®} cells per dose 72 to 96 hours after the initial dose, provided they continue to meet inclusion criteria prior to dosing. Cohort 1d will continue to recruit study subjects until at least 3 eligible subjects have received 2 doses of MultiStem^{®}.
❖ Cohort 2 (MultiStem^{®} product Run-In phase): 50 subjects randomized 1:1 to either MultiStem^{®} therapy (900 million or 1.2 billion cells per dose) or placebo.
❖ Cohort 3 (MultiStem^{®} product): 300 to 400 subjects randomized 1:1 to either MultiStem^{®} therapy (900 million or 1.2 billion cells per dose) or placebo.

In Cohorts 1d, 2, and 3, subjects with persistent or worsening ARDS could receive a second dose of MultiStem^{®} or placebo identical/equivalent to the first dose administered (900 million or 1.2 billion cells per dose or equivalent placebo) 72 to 96 hours after initial dosing of MultiStem^{®} or placebo, provided they continue to meet inclusion criteria prior to dosing; there will be no crossover. Specific study visits for data collection include Day 0 (pre- and post-infusion) and Days 1, 2, 3, 7, 14, 21, 28, 60, 90, 180, and 365. For subjects who receive a second dose of IP, data collection also include Days 4, 5, and 6. Data that are assessed include adverse events (AEs), vital signs, safety laboratory parameters (biochemistry, hematology, and coagulation), COVID-19 test results, respiratory physiologic measures and ventilator settings (PaO2/FiO2 ratio, oxygenation index, peak and plateau pressures, PEEP, and subject position information [i.e., prone, supine, or inclined]), QoL (EuroQoL Five Dimension Questionnaire [EQ-5D]-5L), hospitalization data (ventilator days, Intensive Care Unit [ICU] days, hospitalization days), mortality, Sequential Organ Failure Assessment (SOFA) score, and exploratory biomarkers (white blood cell populations and inflammatory biomarkers).

Aspects and features of the present disclosure are set out in the following numbered clauses which contain the subject matter of the claims of the parent application as filed.
1. A method of treating viral-induced acute respiratory distress syndrome (ARDS) comprising administering to a subject with viral-induced ARDS cells (1) in amounts sufficient, for a time sufficient, and by a route effective to treat the ARDS, wherein said cells (I) are non-embryonic stem, non-germ cells that have undergone at least 10-40 cell doublings in culture and wherein the cells (I) express telomerase and/or oct4, are not transformed, are not tumorigenic and have a normal karyotype.
2. The method of clause 1, wherein the cells (1) express telomerase.
3. The method of either of clause 1 or 2, wherein the cells (I) can differentiate into at least two of the endodermal, ectodermal and mesodermal cell types.
4. The method of any of clauses 1-3, wherein the cells (I) express oct4.
5. The method of any of clauses 1-4, wherein the cells (I) are human.
6. The method of any of clauses 1-5, wherein the cells (1) are derived from bone marrow.
7. The method of any of clauses 1-6, wherein the cells (I) have undergone 40 cell doublings in culture.
8. The method of any of clauses 1-7, wherein the cells (I) can undergo at least 40 cells doublings in culture.
9. The method of any of clauses 1-8, wherein the cells (I) are allogeneic.
10. The method of any of clauses 1-9, wherein the virus that induced the ARDS is *Betacoronavirus.*
11. The method of clause 10, wherein the *Betacoronavirus* is selected from the group consisting of severe acute respiratory syndrome (SARS), Corona Virus or Middle East Respiratory Syndrome (MERS), or severe acute respiratory syndrome Corona Virus 2 (SARS-CoV-2).
12. The method of clause 1, wherein the virus is selected from a group consisting of influenza, coronavirus including (SARS-CoV, MERS-CoV, SARS-CoV2), herpes simplex virus, cytomegalovirus, rhinoviruses, respiratory syncytial virus, parainfluenza virus, human metapneumovirus, and adenovirus.
13. The method of any of clauses 1-12, wherein the subject is human.
14. The method of any of clauses 1-13, wherein the route of the administration is intravenous.

## Claims

1. Cells (I) for use in a method of treating viral-induced acute respiratory distress syndrome (ARDS), comprising administering cells (I) to a subject with viral-induced ARDS, wherein the subject is selected as having a ratio of partial pressure of arterial oxygen to fraction of inspired oxygen (PaO₂/FiO₂) of less than 300 mmHg on positive end-expiratory pressure (PEEP) greater than 5 cm H₂O, and wherein said cells (I) are multipotent adult progenitor cells (MAPCs) **characterised in that** they are non-embryonic, non-germ cells that have undergone at least 10-40 cell doublings in culture and wherein the cells (I) express telomerase and/or oct4, are not transformed, are not tumorigenic and have a normal karyotype.

2. The cells (I) for use according to claim 1, wherein the PaO₂/FiO₂ is less than 200 mmHg on PEEP greater than 5 cm H₂O.

3. The cells (I) for use according to claim 2, wherein the PaO₂/FiO₂ is less than 100 mmHg on PEEP greater than 5 cm H₂O.

4. The cells (I) for use according to any one of claims 1 to 3, wherein the subject has persistent or worsening ARDS.

5. The cells (I) for use according to claim 4, wherein worsening ARDS is defined by the PaO₂/FiO₂ remaining less than 200 mmHg, or not having increased by more than 100 mmHg from a screening assessment.

6. The cells (I) for use according to any one of claims 1 to 5, wherein the subject has a dysregulated immune response, being a maladaptive change in molecular control of immune system processes that leads to disruption of the pulmonary alveolar-capillary barrier, resulting in lung injury **characterised by** hypoxemia, inflammation, and non-cardiogenic pulmonary edema.

7. The cells (I) for use according to any one of claims 1 to 6, wherein the cells (I) are first administered within 48 hours of the subject meeting the diagnostic criteria for ARDS.

8. The cells (I) for use according to any one of claims 1 to 7, wherein the route of administration is intravenous.

9. The cells (I) for use according to any one of claims 1 to 8, wherein the cells (I) are administered as a first dose of from 900 million to 1.2 billion cells (I).

10. The cells (I) for use according to claim 9, wherein a second dose of from 900 million to 1.2 billion cells (I) is administered from 72 to 96 hours after the first dose, optionally wherein the second dose is administered only if the subject has persistent or worsening ARDS.

11. The cells (I) for use according to any one of claims 1 to 10, wherein the cells (I) are co-administered with a further agent selected from an IL-6 inhibitor, an antiviral agent, convalescent plasma and high-dose vitamin C, optionally wherein the IL-6 inhibitor is selected from tocilizumab, siltuximab and sarilumab, and the antiviral agent is selected from remdesivir, favipiravir, and a combination of lopinavir and ritonavir.

12. The cells (I) for use according to any one of claims 1 to 11, wherein the cells (I) express telomerase, can differentiate into at least two of the endodermal, ectodermal and mesodermal cell types, and express oct4.

13. The cells (I) for use according to any one of claims 1 to 12, wherein the cells (I) are human, are derived from bone marrow, and are allogeneic, and wherein the subject is human.

14. The cells (I) for use according to any one of claims 1 to 13, wherein the virus that induced the ARDS is a Betacoronavirus, optionally wherein the Betacoronavirus is severe acute respiratory syndrome (SARS) coronavirus, Middle East respiratory syndrome (MERS) coronavirus, or severe acute respiratory syndrome coronavirus 2 (SARS-CoV-2).

15. The cells (I) for use according to any one of claims 1 to 13, wherein the virus is selected from the group consisting of influenza, coronavirus including SARS-CoV, MERS-CoV and SARS-CoV-2, herpes simplex virus, cytomegalovirus, rhinoviruses, respiratory syncytial virus, parainfluenza virus, human metapneumovirus, and adenovirus.
